# EUROPEAN PATENT SPECIFICATION

(11) **EP 3 796 848 B1**
(45) Date of publication and mention of the grant of the patent: **17.01.2024**
(21) Application number: 19728300.5
(22) Date of filing: 20.05.2019
(51) Int. Cl.: A61B 17/04

(54) **ANCHOR DELIVERY SYSTEMS**
ANKERFREISETZUNGSSYSTEME
SYSTÈME DE DISTRIBUTION D'ANCRAGE

(30) Priority: 23.05.2018 US 201815987040; 18.09.2018 US 201862732712 P
(43) Date of publication of application: 31.03.2021
(73) Proprietor: Smith&Nephew, Inc., Memphis, Tennessee 38116 (US); Smith & Nephew Orthopaedics AG, 6300 Zug (CH); Smith & Nephew Asia Pacific Pte. Limited, Singapore 138565 (SG)
(72) Inventor: REGO, Richard Perry, Memphis, Tennessee 38116 (US); FREEHILL, Michael T., Memphis, Tennessee 38116 (US); TRENHAILE, Scott W., Memphis, Tennessee 38116 (US); LITCHFIELD, Robert B., Memphis, Tennessee 38116 (US); HOUSMAN, Mark Edwin, Memphis, Tennessee 38116 (US); BALBOA, Marc Joseph, Memphis, Tennessee 38116 (US)
(74) Representative: Appleyard Lees IP LLP
(86) International application number: PCT/US2019/033065
(87) International publication number: WO 2019/226520

(56) References cited:
- EP-A1- 0 782 411
- WO-A2-93/10715
- US-A1- 2014 277 129
- US-A1- 2017 156 717

## Description

### TECHNICAL FIELD

The present disclosure relates generally to anchor delivery systems and, more particularly, to anchor driver assemblies for preventing over-insertion of an anchor into bone.

### BACKGROUND

Arthroscopic procedures often require soft tissue to be reattached to bone. To achieve this, anchors are typically placed in the bone and sutures attached to the anchor are passed through the tissue to securely retain the tissue in place. Typical anchors may be pound-in or screw-in type anchors, or combinations of both. However, a combination pound-in/screw-in type anchor can fail during insertion if the threaded, screw-in portion of the anchor is over-inserted by the driver assembly. Furthermore, while some contact between the threads of the anchor and the bone surface is necessary to screw the anchor into the bone, too much axial force can damage the threads, impairing the overall function of the anchor.

Some current driver assemblies, in attempting to control anchor over-insertion, rely on visual markers. These visual markers typically include laser marks on the distal end of the anchor and/or driver. However, these visual markers cannot protect the anchor from damage if they are not clearly visible, or are disregarded by the user. Driver assemblies with internal springs have also been developed to provide axial compliance of the inserter shaft to protect the anchor from over-insertion. However, with higher spring loads, friction between the knob which advances the threaded portion of the anchor and the handle grip may cause the knob to be difficult to turn.

Furthermore, some current anchor systems include of a threaded, internal locking plug advanceable within the pound-in tip for locking the suture within a tip eyelet. To deploy the anchor system in bone, two screwing steps are required for both the screw-in sleeve and the locking plug. Activating the sleeve generally requires twisting a knob at the distal end of a driver handle (that is, the end of the driver handle that is furthest from the user), while screwing in the locking plug is accomplished by turning a knob at a proximal end of the handle. However, in practice, the twisting of the distal knob can counteract the downward force or pressure required to deliver the sleeve into bone. In addition, the distal knob can Attorney Docket No. SN-354PCT (PT-5197-WO-PCT) become obstructed from view by sutures leading out of the body, compromising the user's ability to successfully and repeatedly deliver the sleeve into bone. EP0782411 discloses a spring-loaded reciprocable stylet holder assembly. US 2014/227129 A1 discloses an anchor driver for installing an anchor into bone. The anchor driver includes an outer shaft and a sleeve advancement member for advancing the sleeve as well as an inner shaft and a suture capture advancement member for advancing the suture capture member. The technology also includes a system for tissue repair having an anchor assembly and an anchor driver for installing the anchor assembly into bone.

### SUMMARY

The present invention is defined by the features of the independent claim. Preferred embodiments are defined by the dependent claims.

Described herein is a driver assembly which is configured to protect a threaded sleeve of an anchor from damage due to over-insertion. An internal spring permits axial retraction of the outer shaft, which is coupled to the anchor sleeve, independent of the inner shaft and the handle. Connection members extending along the spring create an axial stop between a drive housing and a center housing of the driver assembly such that the drive housing is allowed to travel proximally in response to axial loading against the spring, while travel distally is limited by the engagement between the connection members and receivers on the center housing. Advantageously, the axial compliance reaction force is internal to the driver assembly, so that rotation of the outer knob is not affected by friction.

Also described herein is a driver assembly in which the knob for screwing the sleeve into bone is located on the proximal half of a driver handle, closer to the user. The sleeve is engaged with and delivered by an outer shaft extending from the handle. An outer shaft hub coupled to the outer shaft is operatively linked to the knob. As such, the user rotates the knob to spin the outer shaft assembly (i.e., the outer shaft hub and the outer shaft) which advances relative to the handle to install the sleeve in bone. Advantageously, the driver assembly of this disclosure is easier and more comfortable to use than current driver assemblies, resulting in a greater likelihood of successful delivery of the sleeve into bone.

Further examples of the driver assemblies of this disclosure may include one or more of the following, in any suitable combination.

In examples, the driver assembly also includes an actuating knob operatively coupled to the inner shaft for moving the inner shaft relative to the outer shaft. In examples, the knob includes a rounded proximal surface. The driver assembly also includes a sleeve advancement member operatively coupled to the outer shaft for moving the outer shaft relative to the inner shaft. In examples, the driver assembly includes a handle grip. At least a portion of the sleeve advancement member and the center housing is disposed within the handle grip. In examples, the handle grip includes a single cleat protruding from the handle grip for managing suture during installation of a tip structure and a threaded member into bone.

In yet further examples, a distal end of the driving housing defines internal threads which are counter-threaded to threads on a surface of the outer shaft hub. The center housing comprises a proximal portion and a distal portion, the distal portion defining the plurality of receivers. In examples, an outer diameter of the center housing is selected to be larger than an outer diameter of the proximal portion of the center housing. A proximal end of the drive housing defines an opening bordered by a plurality of support members extending between the plurality of connection members. The support members are configured to support the spring. In examples, the connection members are defined by an elongated member terminating in a radially-extending foot. In examples, the receivers are in the form of apertures defined by an annular surface of the distal portion of the center housing. In examples, the spring is a compression spring.

Alternative examples of the driver assembly of this disclosure include a handle grip having a proximal end, a distal end and a longitudinal axis extending therebetween. An outer shaft has an open cannulation extending from a proximal end to a distal end. The proximal end of the outer shaft is coupled to an outer shaft hub disposed within the handle grip such that the outer shaft extends from the distal end of the handle grip. An inner shaft is disposed within the outer shaft cannulation and moveable along the longitudinal axis relative to the outer shaft. A proximal end of the inner shaft is operatively coupled to a suture capture knob positioned proximally to the handle grip. A sleeve advancement member is disposed near a proximal end of the handle grip, and a linking mechanism rotationally couples the sleeve advancement member and the outer shaft hub. Rotation of the sleeve advancement member causes the outer shaft to move axially relative to the inner shaft. In further examples, the linking mechanism includes a rod having a first linkage at a proximal end and a second linkage at a distal end. The first linkage is rotationally coupled to the sleeve advancement member and the second linkage rotationally coupled to the outer shaft hub. In other examples, the linking mechanism includes a gear having a first set of teeth for engaging a second set of teeth on the sleeve advancement member and a third set of teeth on the outer shaft hub. In examples, a distal end of the handle grip includes a stationary cap for enclosing the handle grip.

Alternative examples of a driver assembly of this disclosure include a handle assembly including a handle grip. An outer shaft extends from a distal end of the handle assembly. A proximal end of the outer shaft is coupled to an outer shaft hub, and a distal end of the outer shaft is coupled to a proximal anchor body. A sleeve advancement member is at least partially disposed within the handle assembly. A proximal end of the sleeve advancement member defines a first rotatable knob adjacent a proximal end of the handle assembly. A distal end of the sleeve advancement member is operatively coupled to the outer shaft hub such that rotation of the first rotatable knob causes rotational movement of the outer shaft. The rotational movement of the outer shaft is effective to advance the proximal anchor body into bone.

In further examples, the driver assembly includes an anti-rotation member for preventing rotation of the handle grip during rotation of the first rotatable knob. In examples, the outer shaft hub is at least partially disposed within a cavity of a drive housing such that rotation of the first rotatable knob causes rotational movement of the outer shaft hub relative to the drive housing. The cavity of the drive housing includes threads for engaging mating threads on a surface of the outer shaft hub. In examples, the handle assembly includes at least one cleat protruding from the handle grip for providing tension on a suture. In examples, the driver assembly further includes an inner shaft extending through a cannulation of the outer shaft, a proximal end of the inner shaft operatively coupled to a second rotatable knob adjacent the first rotatable knob, a distal end of the inner shaft engaging a plug within a distal anchor body. In examples, the proximal anchor body is a screw-in type anchor. In other examples, the distal anchor body is a pound-in type anchor and includes a transverse eyelet configured for the passage of a suture.

Alternative examples of a driver assembly of this disclosure include a handle assembly including a handle grip. An outer shaft extends from a distal end of the handle assembly. A proximal end of the outer shaft is coupled to an outer shaft hub, and a distal end of the outer shaft is coupled to a proximal anchor body. A sleeve advancement member is at least partially disposed within the handle assembly. A proximal end of the sleeve advancement member defines a first rotatable knob adjacent a proximal end of the handle assembly. A distal end of the sleeve advancement member is operatively coupled to the outer shaft hub. An inner shaft extends through a cannulation of the outer shaft. A proximal end of the inner shaft is operatively coupled to a second rotatable knob adjacent the first rotatable knob such that rotation of the first rotatable knob causes rotational movement of the outer shaft. A distal end of the inner shaft engages a plug within a distal anchor body. The rotational movement of the outer shaft is effective to advance the proximal anchor body into engagement with the distal anchor body.

These and other features and advantages will be apparent from a reading of the following detailed description and a review of the associated drawings. It is to be understood that both the foregoing general description and the following detailed description are explanatory only and are not restrictive of aspects as claimed.

### BRIEF DESCRIPTION OF THE DRAWINGS

The disclosure will be more fully understood by reference to the detailed description, in conjunction with the following figures, wherein:
FIGS. 1A and 1B illustrate of a prior art driver assembly;
FIGS. 2A-E illustrate the component parts of an example of a driver assembly of the present invention;
FIGS. 3A-D illustrate the use of the driver assembly of this disclosure;
FIGS. 4A and 4B show alternative examples of the suture capture knob of this disclosure;
FIGS. 5A-D show alternative examples of the handle grip of this disclosure;
FIGS. 6A-C illustrate an alternative example of the driver assembly not being part of the claimed invention;
   and
FIGS. 7A-C illustrate an alternative example of the driver assembly of FIGS. 6A-C not being part of the claimed invention.
FIGS. 8A-C illustrate the component parts of an alternative example of a driver assembly not being part of the claimed invention; and
FIGS. 9A-D illustrate the use of the driver assembly of FIGS. 8A-C.

### DETAILED DESCRIPTION

In the description that follows, like components have been given the same reference numerals, regardless of whether they are shown in different examples. To illustrate example(s) in a clear and concise manner, the drawings may not necessarily be to scale and certain features may be shown in somewhat schematic form. Features that are described and/or illustrated with respect to one example may be used in the same way or in a similar way in one or more other examples and/or in combination with or instead of the features of the other examples.

As used in the specification and claims, for the purposes of describing and defining the invention, the terms "about" and "substantially" are used to represent the inherent degree of uncertainty that may be attributed to any quantitative comparison, value, measurement, or other representation. The terms "about" and "substantially" are also used herein to represent the degree by which a quantitative representation may vary from a stated reference without resulting in a change in the basic function of the subject matter at issue. "Comprise," "include," and/or plural forms of each are open ended and include the listed parts and can include additional parts that are not listed. "And/or" is open-ended and includes one or more of the listed parts and combinations of the listed parts.

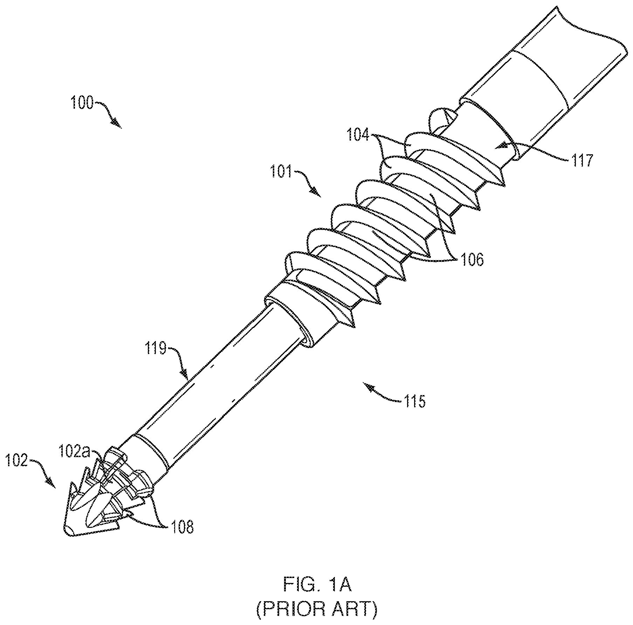

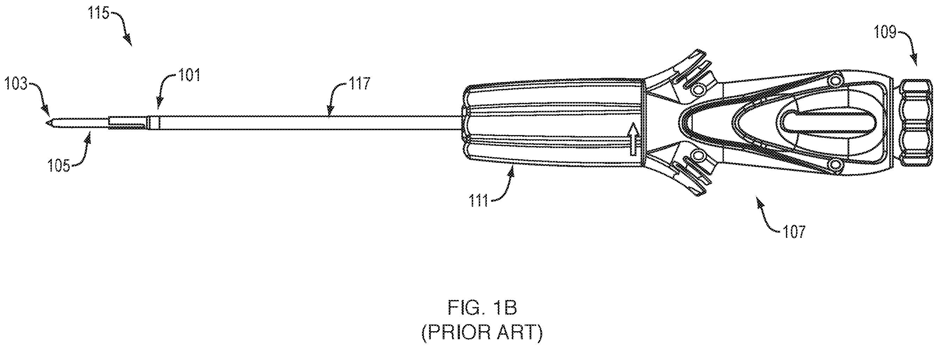

For a better understanding of the current disclosure, FIGS. 1A and 1B depict a prior art suture driver assembly 100 for securing a tissue to bone. As shown in FIG. 1A, the delivery system 100 generally includes a screw-in type anchor sleeve 101 and a pound-in type tip structure 102. In examples, the sleeve 101 includes at least one open helical coil 104 having a plurality of open spacing sections 106 between turns of the open helical coil 104 for allowing bony ingrowth from the bone into an internal volume defined within the open helical coil 104. In examples, the tip structure 102 includes a suture capture member, such as a plug (not shown), advanceable through an internal cavity of the tip structure 102 to lock one or more sutures in an eyelet 102a extending through the tip structure 102. The tip structure 102 can terminate in a pointed and/or barbed tip for breaking through bone during insertion, or can be rounded or flat-ended for various examples where pre-drilling of the bone obviates the need for a distal, terminal point. The tip structure may also include barbs 108 protruding therefrom to improve pullout strength of the tip structure 102. In examples, the sleeve 101 and the tip structure 102 can be constructed from polymers (e.g., PEEK), bioabsorbable materials, metals (e.g., surgical steel, titanium), or any other suitable material.

The threaded anchor sleeve 101 and the tip structure 102 can be installed, for example, into bone using an anchor driver 115, shown in more detail in FIG. 1B. The anchor driver 115 generally includes an outer shaft 117 for engaging with the sleeve 101 or other outer structure, an inner shaft 103 for engaging with the plug of the tip structure 102, and a handle assembly 107 for holding and operating the anchor driver 115. In examples, the anchor driver 115 can also include one or more intermediate shafts 105 to, for example, provide additional stiffness when pounding in the tip structure 102. In examples, the outer shaft 117 may be grooved to engage longitudinal runners (not shown) extending through the internal volume of the sleeve 101. In some examples, the sleeve 101 is screwed or otherwise advanced by the outer shaft 117 into bone over a proximal end of the tip structure 102 by rotation of a sleeve advancement member 111 located at a distal end of the handle assembly 107. In examples, a proximally-located suture capture knob 109 is operatively coupled to a proximal section of the inner shaft 103 such that rotating or twisting the suture capture knob 109 causes the inner shaft 103 to advance the plug within the eyelet 102a of the tip structure 102 (FIG. 1A). Additional non-limiting examples of the delivery system 100 can be found in U.S. Patent No. 9,526,488, to Smith & Nephew, Inc. (Memphis, TN).

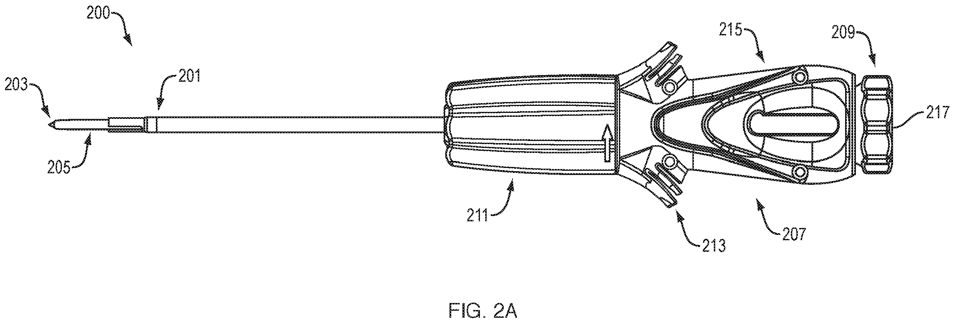

Turning now to FIG. 2A, an example of a driver assembly 200 of this disclosure is shown in a side view. The driver assembly 200 may be used to insert a sleeve, such as sleeve 101, and a tip structure, such as tip structure 102, into bone. Additionally, the driver assembly 200 could be used with any combination device in which the pound-in and screw-in subassemblies can be mechanically decoupled, including devices with guides. The driver assembly 200 could also be used with combination devices in which the pound-in and screw-in phases do not appear to be readily decoupled. Advantageously, the driver assembly 200 of this disclosure is configured such that impact or other axial forces applied to the sleeve while pounding in the tip structure can be alleviated to prevent over-insertion damage, as described in more detail below.

As shown in FIG. 2A, the driver assembly 200 includes an outer shaft 201 for engaging with the sleeve or other threaded structure, an inner shaft 203 extending through the outer shaft 201 for engaging with a tip structure, and a handle assembly 207 for holding and operating the driver assembly 200. In various examples, the driver assembly 200 can also include one or more intermediate shafts 205 to, for example, provide additional stiffness when pounding in a tip structure. The handle assembly 207 can include a suture capture knob 209 for advancing a suture capture member to capture a suture, a sleeve advancement member 211 for screwing in a sleeve, and a handle grip 215 for holding and/or maneuvering the driver assembly 200 during insertion of the anchor into bone. The handle grip 215 can include one or more cleats 213 protruding from the handle grip 215 for placing and/or maintaining tension on a suture during installation of the sleeve and the tip structure into bone. The handle assembly 207 may also include a pounding surface 217 for pounding the tip structure into bone.

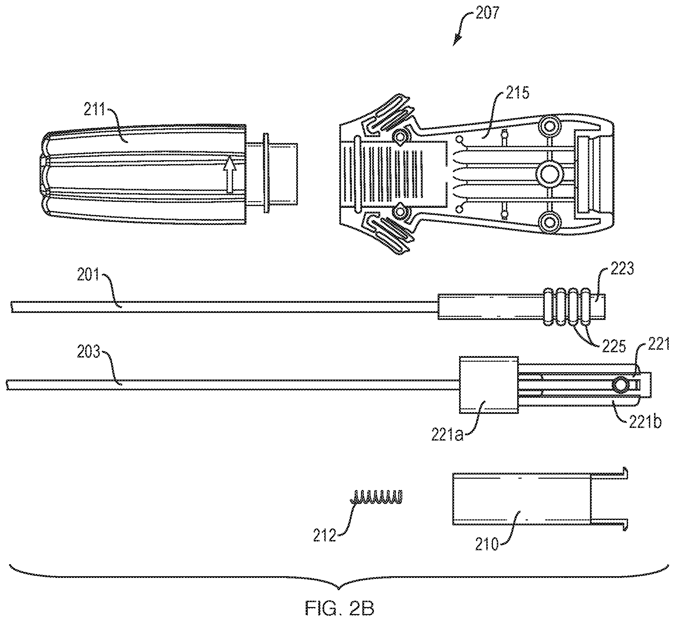

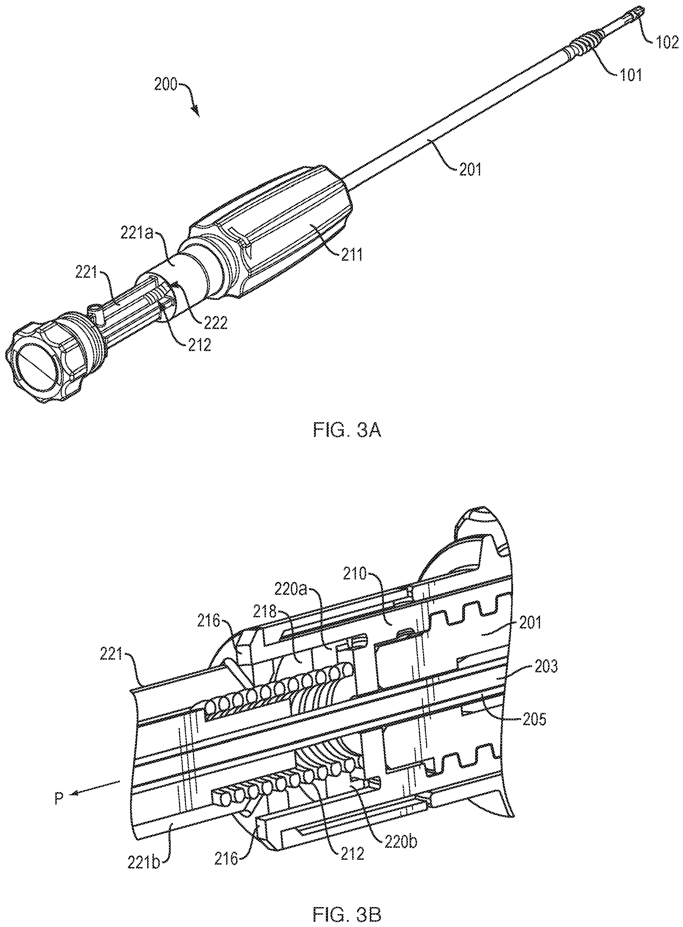

FIG. 2B shows the primary components of the handle assembly 207 of this disclosure in a disassembled view. In particular, FIG. 2B shows the sleeve advancement member 211, a cross-section of the handle grip 215, the outer shaft 201, and the inner shaft 203. A proximal end of the outer shaft 201 is coupled to an outer shaft hub 223 and a proximal end of the inner shaft 203 is coupled to a center housing 221. The center housing 221 includes a distal portion 221a having a larger outer diameter than a grooved proximal portion 221b. In examples, a drive housing 210 defines internal threads (FIG. 3B) which are counter-threaded to threads 225 on the outer shaft hub 223. The drive housing 210 is configured to engage both the sleeve advancement member 211 and the outer shaft hub 223 such that twisting or rotating the sleeve advancement member 211 rotates the outer shaft hub 223. This causes the outer shaft hub 223 to advance distally relative to the center housing 221, consequently advancing the outer shaft 201 relative to the inner shaft 203. This movement in turn causes the sleeve to advance into engagement with the tip structure. The handle assembly 207 also includes a spring 212, the purpose of which will be described in more detail below.

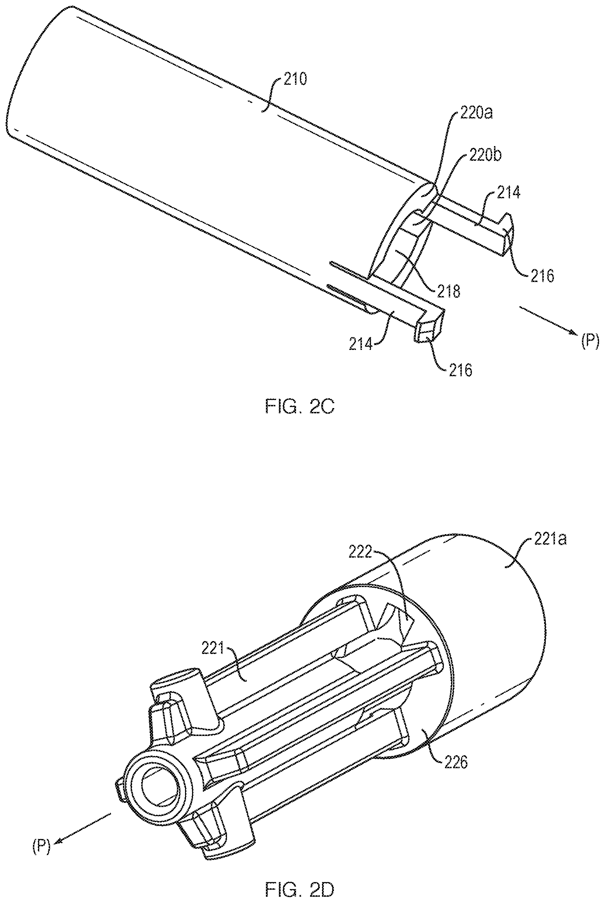

Turning now to FIG. 2C, the drive housing 210 includes a plurality of connection members 214 extending in the proximal direction (P) from a proximal end the drive housing 210. The connection members 214 are part of a mechanism configured to constrain the distal motion of the drive housing 210 with respect to the center housing 221 such that the drive housing 210 and the center housing 221 are not pushed apart by a spring force, while still allowing proximal movement of the drive housing 210 relative to the center housing 221. In FIG. 2C, the connection members 214 are shown in the form of elongated flexures. However, it is contemplated that other types of connection members (such as pins) could be used. Furthermore, while two connection members 214 are shown in FIG. 2C, it will be appreciated that more or fewer than two connection members 214 may be used. In the example of FIG. 2C, each of the connection members 214 terminates in a radially-extending foot 216. The proximal end of the drive housing 210 further defines an opening 218 bordered by semicircular support members 220a, 220b extending between the connection members 214 for supporting the spring 212.

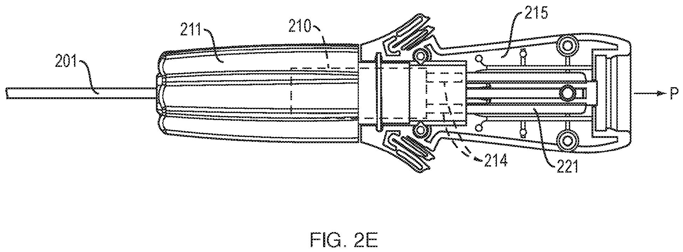

In FIG. 2D, it can be seen that the distal end 221a of the center housing 221 includes a proximally-facing annular surface 226. The annular surface 226 defines a plurality of receivers 222 for engaging the corresponding connection members 214 of the drive housing 210 to limit distal motion of the drive housing 210 relative to the center housing 221. In FIG. 2D, the receivers 222 are shown in the form of apertures configured to engage the feet 216 of the connection members 214, as further described below. However, it should be appreciated that other types of receivers, such as slots, could be used. FIG. 2E illustrates the outer shaft 201, the sleeve advancement member 811, the center housing 221, and the handle grip 815 in an assembled view, with the relative position of the drive housing 210 and connection members 214 indicated.

In various examples, the handle grip 215, the sleeve advancement member 211, the center housing 221, the outer shaft hub 223 and/or the drive housing 210 can each be manufactured from a polymer material and via an injection molding process. However, any other suitable material (e.g., metals, composites, wood) and/or process (e.g., extrusion, machining, electro-chemical machining) can be used. The handle grip 215, the center housing 221, the sleeve advancement member 211, and/or the drive housing 210 can be coupled via an interference fit. However, any other suitable method of coupling (e.g., screws, adhesives, rivets) can be used. The components of the outer shaft 201, the inner shaft 203, and/or the intermediate shaft(s) 205 can be made from a metal material via an extrusion or drawing process. However, any other suitable material (e.g., plastics, composites) and/or process (e.g., injection molding, casting, machining, electro-chemical machining) can be used. The components of the outer shaft 201, the inner shaft 203, and/or the intermediate shaft(s) 205 can be coupled to the handle grip 215, the outer drive hub 223, the center housing 221 and/or the sleeve advancement member 211 via an interference fit. However, any other suitable method of coupling (e.g., screws, adhesives, rivets) can be used.

Turning now to FIG. 3A, in use, various examples of the driver assembly 200 of this disclosure can alleviate impact forces on the sleeve 101 by including the spring 212 within the center housing 221. The spring 212 is configured to allow a relative motion between the outer shaft 201 and the inner shaft 203 (FIG. 3B), thereby absorbing at least a portion of the impact forces exerted on the sleeve 101 during the pounding-in of the tip structure 102. As shown in more detail in FIG. 3B, the spring 212 is positioned between the support members 220a, 220b within the opening 218 of the drive housing 210 such that the spring 212 extends into the proximal portion 221b of the center housing 221 and around the inner shaft 203 (and any intermediate shafts 205). It will be appreciated that, while the spring 212 is depicted to be a compression spring, any suitable spring 212 can be used. Additionally, it is contemplated that any means of introducing compliance between the drive housing 210 and the center housing 221 (for example, foam, rubber, magnet pairs, trapped fluid, flexures, etc.), could be used instead of the spring 212, although not forming part of the claimed invention.

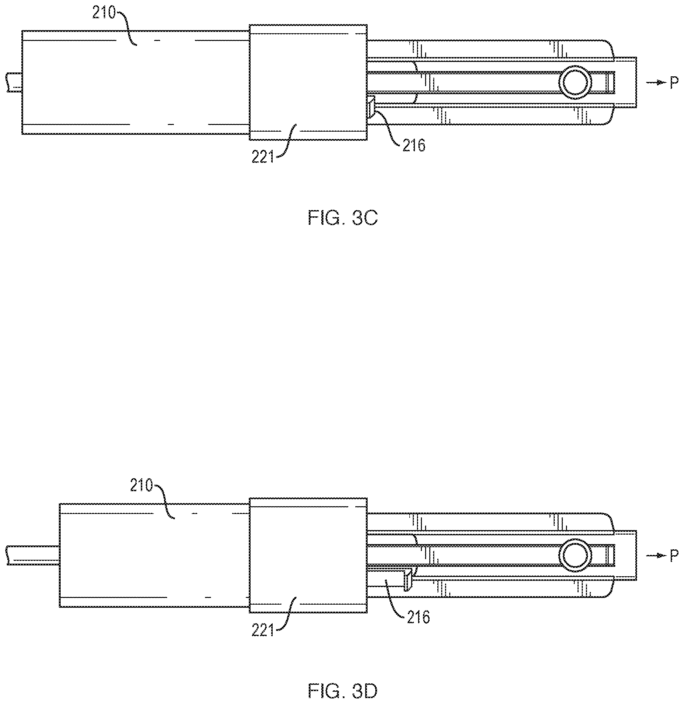

212. Thus, upon an impact or other axial force being applied to the sleeve 101, the outer shaft 201 is configured to move proximally in the direction (P) relative to inner shaft 203. This relative proximal motion of the outer shaft 201 moves the drive housing 210 through the center housing 221 from an initial, first position (FIG. 3C) to an axially-compliant, second position (FIG. 3D), thereby compressing or otherwise actuating the spring 212 and absorbing or otherwise relieving the impact forces. As the drive housing 210 moves proximally through the center housing 221, the receivers 222 in the center housing 221 allow the connection members 214 to travel proximally in response to axial loading against the spring 212. At the same time, travel distally by the drive housing 210 relative to the center housing 221 is limited by engagement of the feet 216 with the receivers 222 in the center housing 221. Thus, the connection members 214 create an axial stop between the drive housing 210 and the center housing 221 to counteract the friction loading that causes the sleeve advancement member 211 to be hard to turn.

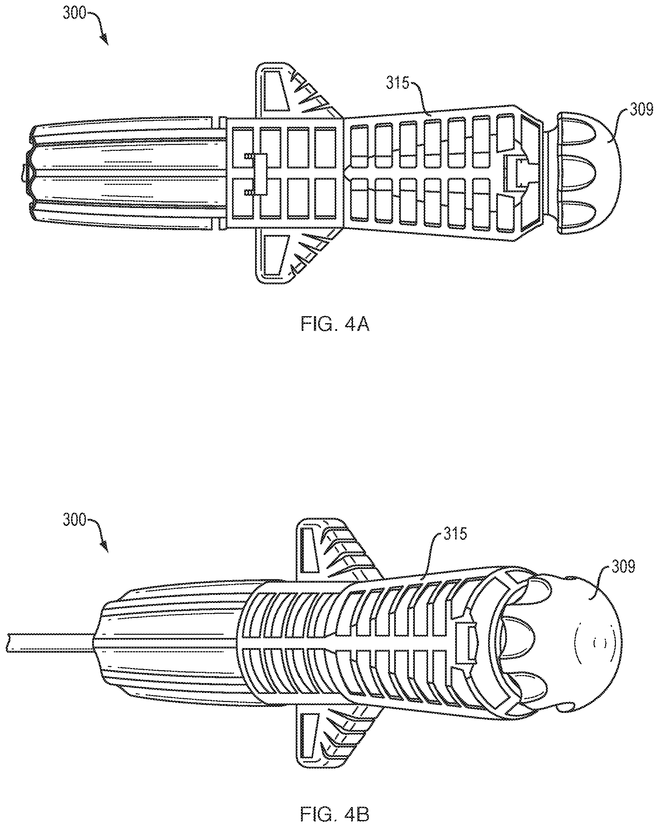

Variations of the suture capture knob 209 will now be discussed with reference to FIGS. 4A and 4B. In FIG. 2A, for example, the suture capture knob 209 is shown as having a flat proximal terminus with an abrupt edge transition toward the pounding surface 217. When the user applies the moderate downward pressure required to use the driver assembly 200, the suture capture knob 209 can press uncomfortably on the palm of the hand. Therefore, it some applications, it may be more convenient to have a suture capture knob 209 with a rounded proximal surface. As shown in FIGS. 4A and 4B, the suture capture knob 309 is rounded proximally and abrupt transitions (i.e., corners) have been eliminated to advantageously make the driver assembly 300 fit more comfortably in the user's hand. In addition, a maximum diameter of the knob 309 is larger than the maximum diameter of the knob 209 to create a smoother transition to the handle grip 315. Thus, the driver assembly 300 is more comfortable for the user when applying moderate downward pressure on the driver assembly 300, which facilitates the threading of the sleeve 101 into bone.

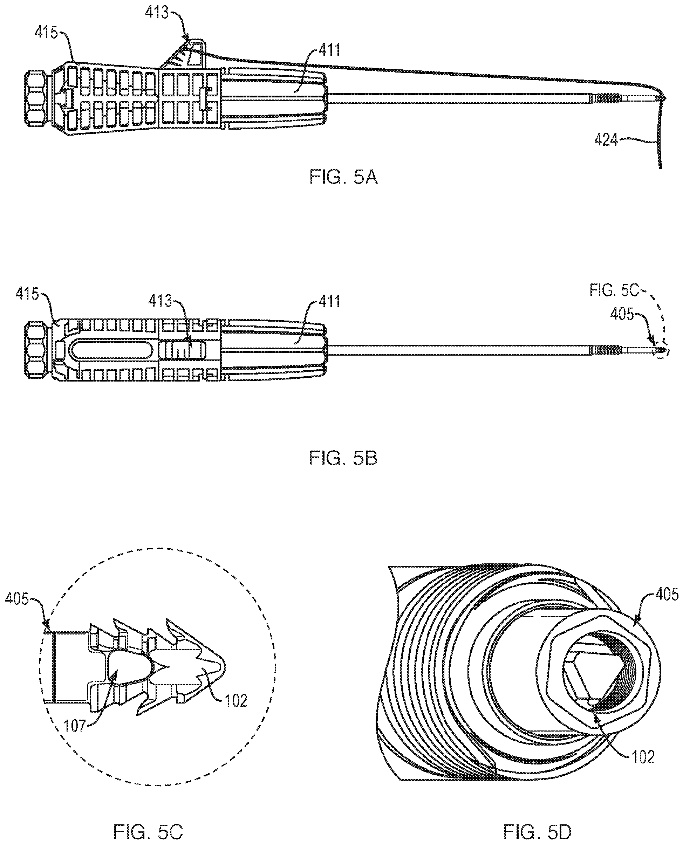

Variations of the handle grip 215 will now be discussed with reference to FIGS. 5A-C. In FIG. 2A, for example, the handle grip 215 is shown as including a pair of symmetrical cleats 213 protruding from the handle grip 215 for placing and/or maintaining tension on a suture during installation of the sleeve 101 and the tip structure 102 into bone. However, in some applications, it may be more convenient to have only one cleat 213 protruding from the handle grip for ease of suture management. As shown in FIGS. 5A and 5B, the handle grip 415 includes a single cleat 413 which is preferentially aligned with a fixed orientation of the eyelet 107 of the tip structure 102 such that suture 424 passed through the eyelet 107 naturally aligns to the single cleat 413. This orientation of the tip structure 102 to the handle grip 415 is controlled by the mating interface between the tip structure 102 and the intermediate shaft 405 (FIG. 5C). For example, the mating interface may include a polygonal mating surface which limits the rotation of the tip structure 102 relative to the intermediate shaft 405, as in FIG. 5C. However, other mating interfaces which limits the rotation of the tip structure 102 relative to the intermediate shaft 405 are contemplated by this disclosure. Advantageously, in addition to simplifying management of the suture 424, having a single cleat 413 allows the sleeve advancement member 411 to be less obscured by the suture 424 and therefore more easily rotated by the user.

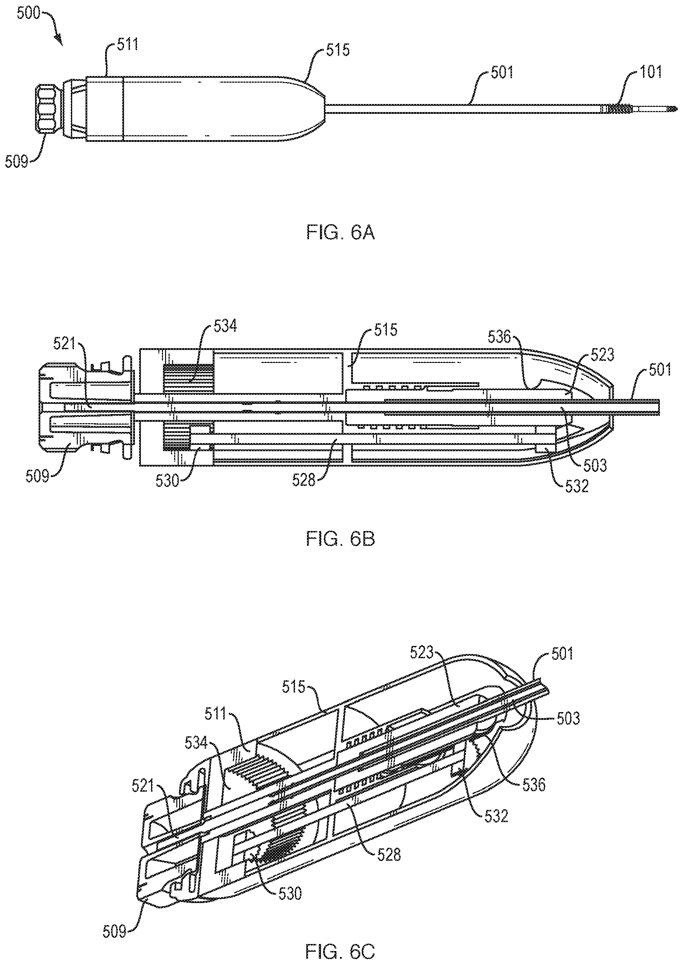

Variations on the driver assembly 200 will now be discussed with regard to FIGS. 6A-C. In FIG. 2A, for example, the sleeve advancement member 211 is shown as being located at a distal end of the handle grip 215. However, in some applications, it may be more convenient to have the sleeve advancement member 211 located closer to the suture capture knob 209 (i.e., towards a proximal end of the handle grip 215) for ease of use. FIG. 6A illustrates an example of a driver assembly 500 in which the sleeve advancement member 511 is located near the suture capture knob 509 towards a proximal end of the handle grip 515. As shown in FIG. 6A, the outer shaft 501 extends from the distal end of the handle grip 515 for engaging the sleeve 101. As illustrated in more detail in FIGS. 6B and 6C, a proximal end of the outer shaft 501 is coupled to an outer shaft hub 523 disposed within the handle grip 515. The inner shaft 503 is axially slidable within a cannulation of the outer shaft 501 relative to the outer shaft 501. A proximal end of the inner shaft 503 is coupled a center housing 521 which is operatively coupled to the suture capture knob 509 positioned proximally to the handle grip 515. As described above, the sleeve advancement member 511 is disposed between the handle grip 515 and the suture capture knob 509.

In order to operatively couple the outer shaft hub 523 (and thus, the outer shaft 501) with the sleeve advancement member 511, a linking mechanism is used to engage the outer shaft hub 523 and the sleeve advancement member 511. In examples, the linking mechanism includes a rod 528 having a first linkage 530 (for example, a first gear) at a proximal end of the rod 528 and a second linkage 532 (for example, a second gear) at a distal end of the rod 528. The first linkage 530 engages a gear 534 which is rotationally coupled to the sleeve advancement member 511, while the second linkage 532 engages a spline 536 on the outer shaft hub 523. Thus, rotation of the sleeve advancement member 511 causes rotation of the outer shaft hub 523, which in turn causes the outer shaft 501 to move axially relative to the inner shaft 503. Notably, while the linking mechanism is shown in FIGS. 6B and 6C as including gears, other rotational linkages, such as bands, belts, or chains, are contemplated by this disclosure.

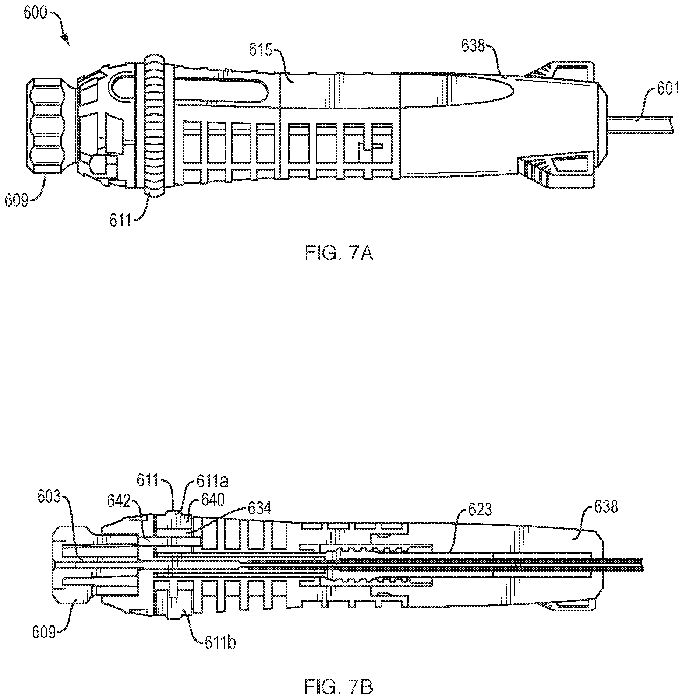

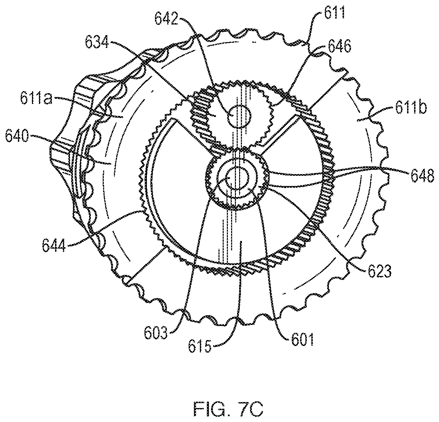

Another variation on the driver assembly 500 will now be discussed with regard to FIGS. 7A-C. FIG. 7A illustrates an alternative example of the driver assembly 600 in which the sleeve advancement member 611 is located near the suture capture knob 609 towards a proximal end of the handle grip 615. As shown in FIG. 7A, a distal end of the handle grip 615 includes a stationary cap 638 for enclosing the handle grip 615. The outer shaft 601 extends from the stationary cap 638 for engaging the sleeve 101. As shown in more detail in FIG. 7B, the sleeve advancement member 611 defines an internal ring gear 640. In examples, the sleeve advancement member 611 is fabricated in two pieces 611a, 611b to facilitate its assembly onto the handle grip 615. An idler gear 634 is mounted on a rod or shaft 642 within the handle grip 615. As shown in FIG. 7C, the internal ring gear 640 includes gear teeth 644 that mate with gear teeth 646 on the idler gear 634 which, in turn, mate with gear teeth or splines 648 on an exterior of the outer shaft hub 623. Thus, rotation of the sleeve advancement member 611 causes rotation of the outer shaft hub 623, which in turn causes the outer shaft 601 to move axially relative to the inner shaft 603 to install the sleeve 101. Notably, while the linking mechanism is shown in FIGS. 7B and 7C as including gears, other rotational linkages, such as bands, belts, or chains, are contemplated by this disclosure.

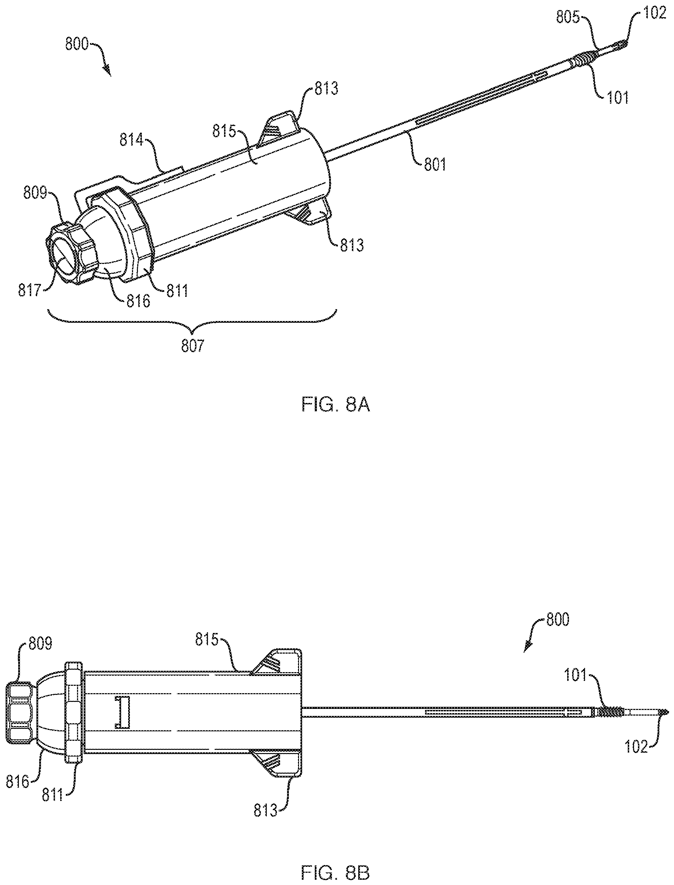

Another variation on the driver assembly 600 will now be discussed with regard to FIGS. 8A and 8B. FIGS. 8A and 8B illustrate an alternative example of the driver assembly 800 in which the sleeve advancement member 811 is located near the suture capture knob 809 towards a proximal end of the handle grip 815. As shown in FIG. 8A, the driver assembly 800 includes an outer shaft 801 for engaging with the sleeve 101 or other threaded structure, at least one middle shaft 105 extending through the outer shaft 801 for engaging with the tip structure 102, and a handle assembly 807 for holding and operating the driver assembly 800. In examples, the handle assembly 807 can also include a center housing 816 for holding the middle shaft 805. The handle assembly 807 also includes a suture capture knob 809 for advancing a plug within the tip structure 102, a sleeve advancement member 811 for screwing in the sleeve 101, and a handle grip 815 for holding and/or maneuvering the delivery system 800 during insertion of the sleeve 101 into bone. Notably, a visible portion of the sleeve advancement member 811 is located adj acent the proximal end of the handle assembly 807. The handle grip 815 can include one or more cleats 813 protruding from the handle grip 815 for placing and/or maintaining tension on a suture threaded through the tip structure 102. The suture capture knob 809 may also include a pounding surface 817 for pounding the tip structure 102 (for example, with a mallet) into bone. Finally, the driver assembly 800 may include an anti-rotation feature, such as a bridge component 814, fixedly coupled to both the center housing 816 and the handle grip 815, the purpose of which will be described in more detail below.

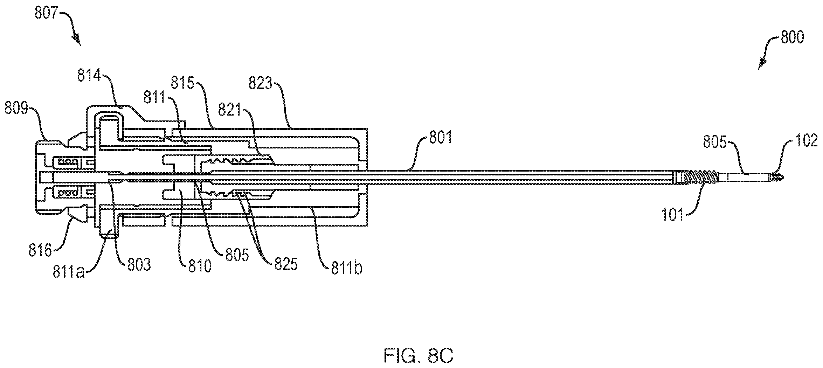

FIG. 8C shows the internal components of the driver assembly 800 of this disclosure in a cross-sectional view. In particular, FIG. 8C shows the sleeve advancement member 811, the handle grip 815, the outer shaft 801, and the inner shaft 803. A proximal end of the outer shaft 801 is coupled to an outer shaft hub 823 disposed within the handle grip 815, while a proximal end of the inner shaft 803 is coupled to the suture capture knob 809. A proximal end 811a of the sleeve advancement member 811 defines a rotatable knob extending outside of the handle grip 815 near the proximal end of the handle assembly 807. In examples, a drive housing 810 disposed within the handle assembly 807 may define a receiving cavity 821 having threads 825 which are counter-threaded with a surface of the outer drive hub 823. The outer drive hub 823 can be further engaged with a distal end 811b of the sleeve advancement member 811. Thus, twisting or rotating the sleeve advancement member 811 via the rotatable knob rotates the outer drive hub 823, thereby causing the outer drive hub 823 to advance distally through the drive housing 810 and, consequently, to advance the sleeve 101 into engagement with bone and/or the tip structure 102. Meanwhile, the bridge component 814 stabilizes the handle grip 815 to the center housing 816, preventing the handle grip 815 from rotating during rotation of the sleeve advancement member 811.

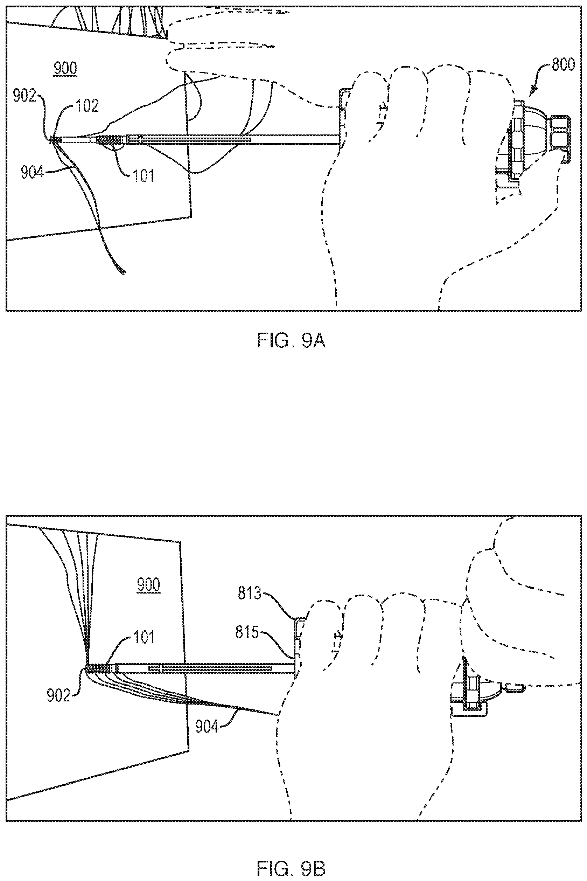

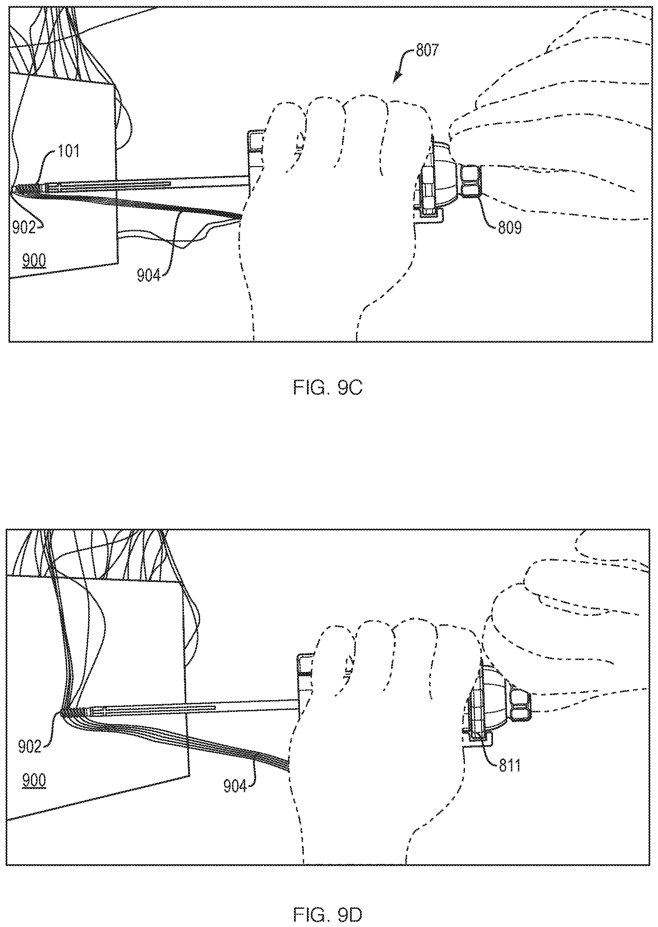

Turning now to FIGS. 9A-D, use of the driver assembly 800 of this disclosure is illustrated during installation of a sleeve 101 into bone 900. The installation procedure can include drawing one or more sutures 904 through a soft tissue and placing at least one end of the sutures 904 through the eyelet of the tip structure 102. In FIG. 9A, the driver assembly 800 is shown as held by a user as it would be when the driver assembly 800 is first delivered into bone 900, i.e., with the tip structure 102 positioned adjacent a bone hole 902 and one or more sutures 904 extending therefrom. In FIG. 9B, the tip structure 102 is placed at the bottom of the bone hole 902, bringing the sleeve 101 into a predetermined proximity of the bone 900 (e.g., adjacent to the bone 900 at a nominal clearance from the bone 900). The sutures 904 are then pulled to the desired tension and attached to the cleats 813 on the handle grip 815. As shown in FIG. 9C, the suture capture knob 809 is then rotated, for example, until a feedback mechanism of a torque and/or travel limiter of the handle assembly 807 indicates full advancement. Such rotation serves to advance the locking plug into the eyelet of the tip structure 102 to advantageously lock the sutures 904 within the tip structure 102 independent of the compression fit between the sleeve 101 and the bone 900. Finally, as shown in FIG. 9D, the sleeve 101 is then screwed into the bone hole 902 via rotation of the sleeve advancement member 811, which moves the sleeve 101 axially and engages the distal end of the sleeve 101 with the proximal end of the tip structure 102, further locking the suture 904 between the sleeve 101 and the bone 900. Optionally, the tip structure 102 can be placed within bone, ends of the suture 904 placed through the soft tissue, and the ends of the suture 904 then placed through the eyelet of the tip structure 102. In other examples, tension on the sutures 904 can be temporarily released by retracting the plug so that, for example, the user can re-tension the sutures 904.

## Claims

1. A driver assembly (200, 300) comprising:
an outer shaft (201) having a proximal end and a distal end, the proximal end of the outer shaft (201) coupled to an outer shaft hub (223);
an inner shaft (203) disposed within a cannulation of the outer shaft (201) and axially slidable relative to the outer shaft (201);
a center housing (221) coupled to a proximal end of the inner shaft (203);
a drive housing (210) coupled to the outer shaft hub (223), the drive housing (810) at least partially receivable within an opening in the center housing (221) and longitudinally slidable relative to the center housing (221), the drive housing (210) having a plurality of proximally-extending connection members (214) engageable with a plurality of corresponding receivers (222) on the center housing (221); and
a spring (212) at least partially disposed between the center housing (221) and the drive housing (210);
wherein the drive housing (210) is configured to travel proximally relative to the center housing (221) in response to axial loading against the spring (212), while distal travel of the drive housing (210) relative to the center housing (221) is limited by the engagement between the connection members (214) and the receivers (222)
wherein a proximal end of the drive housing (210) defines an opening (218) bordered by a plurality of support members (220a, 220b) extending between the plurality of connection members (214), the support members configured to support the spring (212)
**characterised in that** the spring (212) is positioned between the support members (220a, 220b) within the opening (218) of the drive housing (210) such that the spring (212) extends into the proximal portion (221b) of the center housing (221) and around the inner shaft (203).

2. The assembly (200, 300) of claim 1, wherein the distal end of the outer shaft (201) is configured to engage a threaded member (101).

3. The assembly (200, 300) of claim 1, wherein a distal end of the inner shaft (203) is configured to engage a tip structure (102).

4. The assembly (200, 300) of claim 1, further comprising an actuating knob (209) operatively coupled to the inner shaft (203) for moving the inner shaft (203) relative to the outer shaft (201), the knob comprising a rounded proximal surface.

5. The assembly (200, 300) of claim 1, further comprising a sleeve advancement member (211) operatively coupled to the outer shaft (201) for moving the outer shaft (201) relative to the inner shaft (203).

6. The assembly (200, 300) of claim 1, wherein a distal end of the drive housing defines internal threads which are counter-threaded to threads on a surface of the outer shaft hub (223).

7. The assembly (200, 300) of claim 1, wherein the center housing (221) comprises a proximal portion and a distal portion, the distal portion defining the plurality of receivers (222) defined by an annular surface of the distal portion of the center housing (221).

8. The assembly (200, 300) of claim 1, wherein the connection members (214) are defined by an elongated member terminating in a radially-extending foot (216).

## Patentansprüche

1. Treiberanordnung (200, 300), umfassend:
eine äußere Welle (201) mit einem proximalen Ende und einem distalen Ende, wobei das proximale Ende der äußeren Welle (201) mit einer äußeren Wellennabe (223) gekoppelt ist;
eine innere Welle (203), die in einer Bohrung der äußeren Welle (201) angeordnet und relativ zu der äußeren Welle (201) axial verschiebbar ist;
ein mittleres Gehäuse (221), das mit einem proximalen Ende der inneren Welle (203) gekoppelt ist;
ein Antriebsgehäuse (210), das mit der äußeren Wellennabe (223) gekoppelt ist, wobei das Antriebsgehäuse (810) zumindest teilweise in einer Öffnung in dem mittleren Gehäuse (221) aufnehmbar und relativ zu dem mittleren Gehäuse (221) in Längsrichtung verschiebbar ist, wobei das Antriebsgehäuse (210) eine Mehrzahl von sich proximal erstreckenden Verbindungselementen (214) aufweist, die mit einer Mehrzahl von entsprechenden Aufnahmen (222) an dem mittleren Gehäuse (221) in Eingriff bringbar sind; und
eine Feder (212), die zumindest teilweise zwischen dem mittleren Gehäuse (221) und dem Antriebsgehäuse (210) angeordnet ist;
wobei das Antriebsgehäuse (210) dazu ausgelegt ist, relativ zu dem mittleren Gehäuse (221) in Reaktion auf eine axiale Belastung gegen die Feder (212) proximal zu verfahren, während ein distaler Verfahrweg des Antriebsgehäuses (210) relativ zu dem mittleren Gehäuse (221) durch den Eingriff zwischen den Verbindungselementen (214) und den Aufnahmen (222) begrenzt ist,
wobei ein proximales Ende des Antriebsgehäuses (210) eine Öffnung (218) definiert, die durch eine Mehrzahl von Stützelementen (220a, 220b) umrandet ist, die sich zwischen der Mehrzahl von Verbindungselementen (214) erstrecken, wobei die Stützelemente dazu ausgelegt sind, die Feder (212) zu stützen,
**dadurch gekennzeichnet, dass** die Feder (212) zwischen den Stützelementen (220a, 220b) in der Öffnung (218) des Antriebsgehäuses (210) derart positioniert ist, dass die Feder (212) sich in den proximalen Abschnitt (221b) des mittleren Gehäuses (221) und um die innere Welle (203) erstreckt.

2. Anordnung (200, 300) nach Anspruch 1, wobei das distale Ende der äußeren Welle (201) dazu ausgelegt ist, ein Gewindeelement (101) in Eingriff zu bringen.

3. Anordnung (200, 300) nach Anspruch 1, wobei ein distales Ende der inneren Welle (203) dazu ausgelegt ist, eine Spitzenstruktur (102) in Eingriff zu bringen.

4. Anordnung (200, 300) nach Anspruch 1, ferner umfassend einen Betätigungsknopf (209), der mit der inneren Welle (203) zum Bewegen der inneren Welle (203) relativ zu der äußeren Welle (201) wirkgekoppelt ist, wobei der Knopf eine gerundete proximale Fläche umfasst.

5. Anordnung (200, 300) nach Anspruch 1, ferner umfassend ein Hülsenvorschubelement (211), das mit der äußeren Welle (201) zum Bewegen der äußeren Welle (201) relativ zu der inneren Welle (203) wirkgekoppelt ist.

6. Anordnung (200, 300) nach Anspruch 1, wobei ein distales Ende des Antriebsgehäuses Innengewinde definiert, die Gegengewinde zu Gewinden auf einer Fläche der äußeren Wellennabe (223) sind.

7. Anordnung (200, 300) nach Anspruch 1, wobei das mittlere Gehäuse (221) einen proximalen Abschnitt und einen distalen Abschnitt umfasst, wobei der distale Abschnitt die Mehrzahl von Aufnahmen (222) definiert, die durch eine ringförmige Fläche des distalen Abschnitts des mittleren Gehäuses (221) definiert sind.

8. Anordnung (200, 300) nach Anspruch 1, wobei die Verbindungselemente (214) durch ein längliches Element definiert sind, das in einem sich radial erstreckenden Fuß (216) endet.

## Revendications

1. Ensemble d'entraînement (200, 300) comprenant :
un arbre externe (201) ayant une extrémité proximale et une extrémité distale, l'extrémité proximale de l'arbre externe (201) étant accouplée à un moyeu d'arbre externe (223) ;
un arbre interne (203) disposé à l'intérieur d'une canule de l'arbre externe (201) et pouvant coulisser axialement par rapport à l'arbre externe (201) ;
un boîtier central (221) accouplé à une première extrémité de l'arbre interne (203) ;
un boîtier d'entraînement (210) accouplé au moyeu d'arbre externe (223), le boîtier d'entraînement (810) pouvant être au moins partiellement reçu dans une ouverture du boîtier central (221) et pouvant coulisser longitudinalement par rapport au boîtier central (221), le boîtier d'entraînement (210) ayant une pluralité d'éléments de liaison à extension proximale (214) pouvant venir en prise avec une pluralité de récepteurs (222) correspondants sur le boîtier central (221) ; et
un ressort (212) au moins partiellement disposé entre le boîtier central (221) et le boîtier d'entraînement (210) ;
le boîtier d'entraînement (210) étant conçu pour se déplacer de manière proximale par rapport au boîtier central (221) en réponse à une charge axiale contre le ressort (212), tandis que le déplacement distal du boîtier d'entraînement (210) par rapport au boîtier central (221) est limité par la mise en prise entre les éléments de liaison (214) et les récepteurs (222)
une extrémité proximale du boîtier d'entraînement (210) définissant une ouverture (218) bordée par une pluralité d'éléments de support (220a, 220b) s'étendant entre la pluralité d'éléments de liaison (214), les éléments de support étant conçus pour soutenir le ressort (212)
**caractérisé en ce que** le ressort (212) est positionné entre les éléments de support (220a, 220b) à l'intérieur de l'ouverture (218) du boîtier d'entraînement (210) de sorte que le ressort (212) s'étende dans la partie proximale (221b) du boîtier central (221) et autour de l'arbre interne (203).

2. Ensemble (200, 300) selon la revendication 1, l'extrémité distale de l'arbre externe (201) étant conçue pour venir en prise avec un élément fileté (101).

3. Ensemble (200, 300) selon la revendication 1, une extrémité distale de l'arbre interne (203) étant conçue pour venir en prise avec une structure de pointe (102).

4. Ensemble (200, 300) selon la revendication 1, comprenant en outre un bouton d'actionnement (209) accouplé fonctionnellement à l'arbre interne (203) pour déplacer l'arbre interne (203) par rapport à l'arbre externe (201), le bouton comprenant une surface proximale arrondie.

5. Ensemble (200, 300) selon la revendication 1, comprenant en outre un élément d'avancement de manchon (211) accouplé fonctionnellement à l'arbre externe (201) pour déplacer l'arbre externe (201) par rapport à l'arbre interne (203).

6. Ensemble (200, 300) selon la revendication 1, une extrémité distale du boîtier d'entraînement définissant des filets internes qui sont contre-filés à des filets sur une surface du moyeu d'arbre externe (223).

7. Ensemble (200, 300) selon la revendication 1, le boîtier central (221) comprenant une partie proximale et une partie distale, la partie distale définissant la pluralité de récepteurs (222) définis par une surface annulaire de la partie distale du boîtier central (221).

8. Ensemble (200, 300) selon la revendication 1, les éléments de liaison (214) étant définis par un élément allongé se terminant par un pied s'étendant radialement (216) .
